# EUROPEAN PATENT SPECIFICATION

(11) **EP 3 197 394 B1**
(45) Date of publication and mention of the grant of the patent: **24.07.2019**
(21) Application number: 15843924.0
(22) Date of filing: 23.09.2015
(51) Int. Cl.: A61F 2/07, A61F 2/89, A61F 2/82, A61F 2/848, A61F 2/915

(54) **ENDOPROSTHESIS WITH PREDETERMINED CURVATURE FORMED BY TETHERS**
ENDOPROTHESE MIT VORDEFINIERTER KRÜMMUNG DURCH FACHGURTE
ENDOPROTHÈSE AYANT UNE COURBURE PRÉDÉFINIE FORMÉE PAR ATTACHES

(30) Priority: 23.09.2014 US 201414494136
(43) Date of publication of application: 02.08.2017
(73) Proprietor: Cardinal Health Switzerland 515 GmbH, 6340 Baar (CH)
(72) Inventor: MAJERCAK, David C., Femont, California 94555 (US)
(74) Representative: Wallace, Sheila Jane
(86) International application number: PCT/US2015/051575
(87) International publication number: WO 2016/049102

(56) References cited:
- WO-A1-03/034948
- WO-A1-2009/102441
- AU-B1- 2010 201 676
- US-A- 4 629 458
- US-A- 5 873 906
- US-A1- 2008 039 926
- US-A1- 2013 261 727
- US-B2- 7 615 072
- US-B2- 7 794 492
- US-B2- 8 083 792

## Description

### Background

An aneurysm is an abnormal dilation of a layer or layers of an arterial wall, usually caused by a structural defect due to hardening of the artery walls or other systemic defects such as aortic dissection due to high blood pressure. In the aorta leading into the heart, a thoracic aortic aneurysm (TAA) may occur when the arterial wall of the thoracic aorta is weakened due to the pressure of the blood being pumped by the heart. The TAA is typically presented as a large swelling or bulge under a chest X-ray or ultrasound. When left untreated, the aneurysm may rupture, usually causing rapid fatal hemorrhaging.

As is the case with abdominal aortic aneurysms, the widely accepted approach to treating an aneurysm in the thoracic aorta is surgical repair, involving replacing the aneurysmal segment with a prosthetic device. This surgery, as described above, is a major undertaking, with associated high risks and with significant mortality and morbidity.

One alternative to the surgical repair is to use an endovascular procedure, i.e., catheter directed, techniques for the treatment of aneurysms, specifically for TAA. This has been facilitated by the development of vascular stents, which can and have been used in conjunction with standard or thin-wall graft material in order to create a stent-graft or endograft. The potential advantages of less invasive treatments have included reduced surgical morbidity and mortality along with shorter hospital and intensive care unit stays.

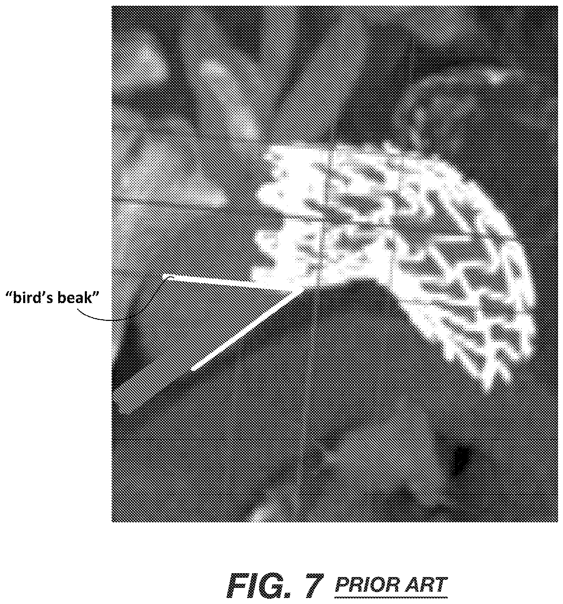

One concern with the use of TAA is the prominence of endoleaks arising from a lack of apposition of a stent-graft to the aortic wall along the inside curve of the aorta. This is believed to be caused by a "bird-beak" (shown here in Figure 7) in a radiologic image of the stent-graft in the aortic arch. In brief, the bird-beak is typically a triangulated wedge between the outside surface of the stent-graft and the inside surface of the aortic wall. The bird-beak is believed to lead endoleaks and the disruption of the normal fluid dynamics of the vasculature as described by F. Auricchio et al., "Patient-specific analysis of post-operative aortic hemodynamics: a focus on thoracic endovascular repair (TEVAR)" published January 24, 2014.

WO03034948 discloses a prosthetic device for the carriage of fluids within a human or animal body and to be placed in or replace a curved lumen. The prosthetic device has a control arrangement to control the length of one side with respect to the other side so that the device can be curved insitu to fit the curved lumen. The control arrangement can be an expansion restriction arrangement or a length reduction arrangement.

### Summary of the Disclosure

Accordingly, I have devised an improved endoprosthesis that is believed to be heretofore not available in the prior art. My improvement is an endoprosthesis for repair of aneurysms. In particular, a thoracic endovascular implant is provided that includes a generally tubular graft, a plurality of stent hoops and at least one suture. The generally tubular graft extends along a longitudinal axis from a first opening to a second opening spaced apart along the longitudinal axis. The plurality of stent hoops is attached to the graft and disposed between the first opening and the second opening of the graft to define a stent graft. Each of the stent hoops has a sinusoidal configuration disposed about the longitudinal axis with apices spaced apart along the longitudinal axis. The apices of one stent hoop are spaced apart at a predetermined distance along the longitudinal axis from adjacent apices of another stent hoop. The at least one suture connects one apex of one stent hoop disposed on the graft to two apices of another stent hoop disposed on the graft to reduce the predetermined distance so that in a compressed or crimped configuration (as inside a catheter sheath prior to delivery in a vessel), the stent-graft extends generally linearly as with the typical stent-graft. Yet in a released configuration (unconstrained in a catheter sheath) in a body vessel, the stent-graft is self-adjusting in-situ so as to curve away from the longitudinal axis to conform to the body vessel and reduce formation of a gap between one end of the stent-graft with an inner surface of the body vessel.

In addition to the embodiments described above, other features recited below can be utilized in conjunction therewith. For example, the at least one suture comprises three sutures in which each suture connects one apex of one stent hoop to two apices of another stent hoop; the one apex of one stent hoop is disposed between two apices of another stent hoop; the
stent-graft is curved along a radius of about 3 centimeters. the radius of curvature defines an arcuate portion of a virtual circle, wherein the arcuate portion includes an angle of approximately 45 degrees; the generally tubular graft comprises a synthetic material selected from a group consisting of nylon, ePTFE, PTFE, Dacron and combinations thereof; the generally tubular graft comprises a generally constant inside diameter smaller than an outside diameter of the stent hoop; the generally tubular graft comprises at least one flared end; the plurality of stent hoops are disposed on the inside surface of the stent-graft; the predetermined distance comprises a distance selected from any value between about 1 mm to about 2 mm; another stent hoop configured with retention barbs is connected to a cranial end of the graft.

### Brief Description of the Figures

The foregoing and other features and advantages of the invention will be apparent from the following, more particular description of preferred embodiments of the invention, as illustrated in the accompanying drawings.
Figure 1A illustrates an exemplary implant for TAA that is shown in its constrained or undeployed configuration inside a delivery catheter;
Figure 1B illustrates a stent hoop used in the cranial portion of the implant;
Figure 1C illustrates a stent hoop used in the body of the implant;
Figure 2 illustrates the implant of Figure 1A in a fully deployed or unconstrained configuration;
Figure 3 is a close-up of the tri-tether connections used in Figure 2;
Figure 4 is a plan view of a prototype of Figure 2;
Figure 5 illustrate yet another embodiment of the implant in Figure 1A;
Figure 6 illustrates yet another implant of Figure 1A;
Figure 7 is a close-up radiographic image of a known stent-graft used for TAA.

The accompanying drawings, which are incorporated herein and constitute part of this specification, illustrate presently preferred embodiments of the invention, and, together with the general description given above and the detailed description given below, serve to explain features of the invention (wherein like numerals represent like elements).

### Modes of Carrying Out the Invention

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±50% of the recited value, e.g. "about 50%" may refer to the range of values from 51% to 99%. In addition, as used herein, the terms "patient," "host," "user," and "subject" refer to any human or animal subject and are not intended to limit the systems or methods to human use, although use of the subject invention in a human patient represents a preferred embodiment. The uses of the terms "cranial" or "caudal" are in this application are used to indicate a relative position or direction with respect to the person receiving the implant. As applied to "cranial," the term indicates a position or direction closer to the heart, while the term "caudal" indicates a position or direction further away from the heart of such a subject.

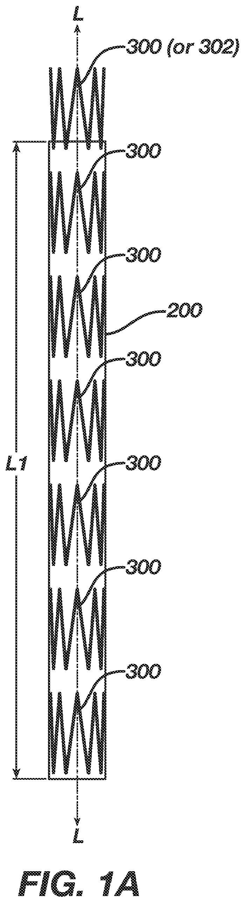

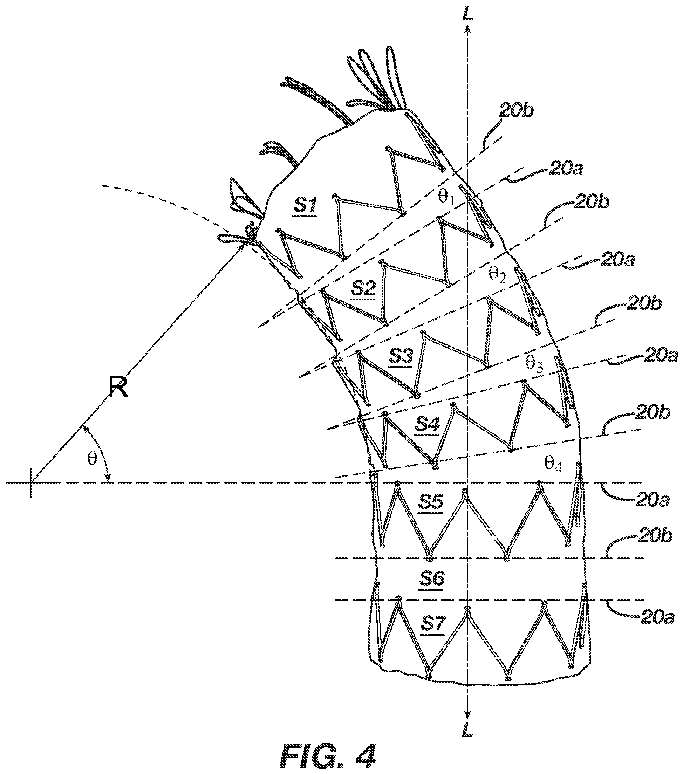

An endovascular implant 100 that can be used in a thoracic aortic aneurysm is shown in Figure 1A. Implant 100 includes three components: a graft 200, stent hoops 300, and sutures 400. As shown in Figure 1A, the implant 100 is in a constrained state such as in a delivery catheter prior to deployment. In this first state, the implant 100 has a small outer diameter while being constrained to a linear configuration. In the unconstrained (or expanded) state in which the implant 100 is unsupported, shown here in Figure 4, the implant 100 takes on a curvilinear configuration, automatically (by virtue of this invention), in which a portion of the implant is linear and another portion is generally curved. Thus, the advantage of my invention is the ability to be constrained so as to conform to a linear configuration while in a catheter but yet when unconstrained, the implant 100 takes on a predetermined curvilinear configuration that mitigates or virtually the drawbacks of the formation of a "bird's beak" in the known TAA stent-graft shown in Fig. 8.

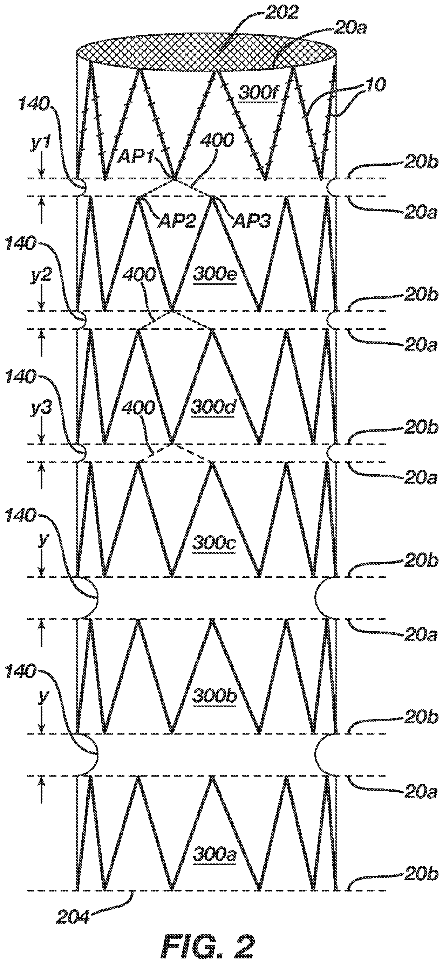

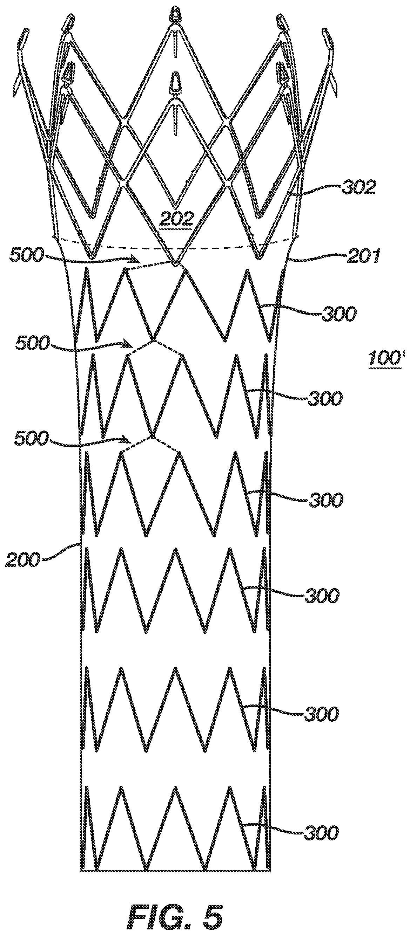

Referring back to Figure 2, the graft 200 can be a generally tubular graft 200 that extends along a longitudinal axis L-L from a first opening 202 to a second opening 204 spaced apart along the longitudinal axis L-L. The graft 200 may be formed from a suitable synthetic material that is biocompatible with physiological fluids. In particular, the material of graft 200 is selected from a group primarily of nylon, ePTFE, PTFE, Dacron and combinations thereof. In one embodiment, the generally tubular graft 200 may have a generally constant inside diameter. Alternatively, the graft 200 may include at least one flared end portion 201 (Fig. 5) as part of implant 100'. Prior to attachment of the graft component to the stent hoops, crimps are formed between the stent positions by placing the graft material on a shaped mandrel and thermally forming indentations in the surface. In the exemplary embodiment illustrated in Figure 2, the crimped grooves 140 are from about one millimeter ("mm") to about two mm long and 0.5 mm deep. With these dimensions, the endovascular graft can bend and flex while maintaining an open lumen. Also, prior to attachment of the graft material to the stent hoops, the graft material is cut in a shape to conform to the shapes of the stent hoops. In one exemplary embodiment, the fabric for the graft material is a forty denier (denier is defined in grams of nine thousand meters of a filament or yarn), twenty-seven filament polyester yarn, having about seventy to one-hundred end yarns per cm per face and thirty-two to forty-six pick yarns per cm face. At this weave density, the graft material is relatively impermeable to blood flow through the wall, but is relatively thin, ranging from between approximately 0.08 to approximately 0.12 mm in wall thickness.

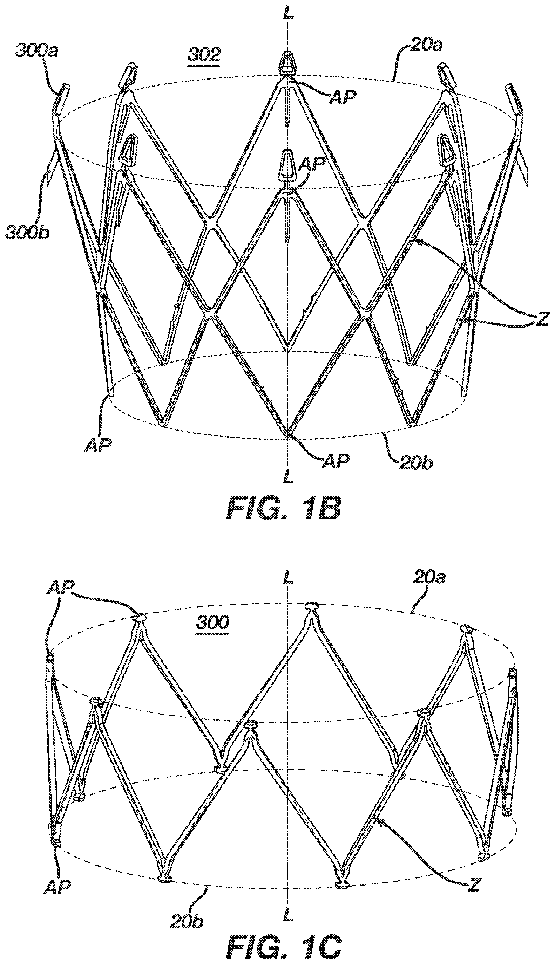

As shown diagrammatically in Figure 2, the plurality of stent hoops 300 (designated as 300a-300f, from a caudal end to the cranial end) are attached to the graft 200 to define stent-graft 100 (including 100' and 100"). The stent hoops 300 can be disposed on the outside surface of the graft 200. In the preferred embodiments, the stent hoops 300 are disposed on the inside surface of the graft 200 and attached with suture retainer 10 or adhesives. It is to be understood that retainer 10 (in the form of adhesive or sutures) is used in the remainder of the support hoops 300a-300e. To ensure sufficient radial expansion force for support of the inner surface of body vessel, the stent hoop 300 may have an outside diameter greater than the inside diameter of the graft. At a distal end of the stent graft 100, a stent hoop 300 (or 302) to can act as an anchor by having a portion of the stent hoop attached to the graft 200. Where increased retention to a body vessel (e.g., in the thoracic artery) is desired, a stent hoop 302 with barbs or hooks 300b (Fig. 1C) can be provided. The configuration of stent hoop 302 allows for the hooks 302b to be retracted prior to delivery into the body vessel by virtue of the eyelets 300a. Details of the stent hoop 302 are provided in US Patent Publication No. 2011/0071614 filed on September 24, 2009.

Referring to Figures 1B and 1C, each of the stent hoops 300 (or a combination of stent hoops 300 and 302) may have a sinusoidal or zig-zag configuration (as indicated by the dashed line Z) disposed about the longitudinal axis L-L. The zig-zag configuration Z of each stent hoop provides for apices AP that are spaced apart along the longitudinal axis L-L. As shown in Figures 1B and 1C, the apices AP of each hoop (300 or 302) define two respective spaced apart circumferences 20a and 20b about the longitudinal axis L-L.

Referring back to Figure 2, the circumference (20a or 20b) defined by the apices AP of one stent hoop 300 are then spaced apart to a circumference (20b or 20a) defined by the apices of another stent hoop 300 at a predetermined distance y along the longitudinal axis L-L. This separation distance y between each separate stent hoop 300 to adjacent stent hoop 300 can be seen for caudal stent hoops 300a and 300b at the bottom of Fig. 2. For stent hoops 300a and 300b, the hoops are not connected directly to each other but via the graft 100. However, for the remaining stent hoops 300c, 300d, 300e and 300f proximate the cranial end, at least one suture 400 is provided to connect one apex (AP1) of one stent hoop (300f) to two apices (e.g.., AP2 and AP3) of another stent hoop (300e).

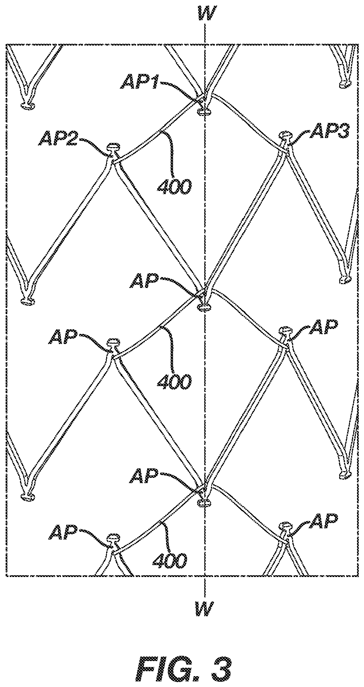

As can be seen in Figures 2 and 3, this additional connection reduces the predetermined distance y to a smaller magnitude (e.g., y1, y2, y3 ...) so that at least one stent hoop (and by virtue of the stent hoop being secured to the graft via retainer suture 10), the stent-graft 100 is pulled away from the longitudinal axis L-L. This allows the graft 100 (Fig. 4) to curve away from the longitudinal axis L-L. Depending on the distance y1, y2 or y3, the stent-graft 100 can conform closely to the body vessel and reduce the formation of a gap (i.e., the bird's beak shown in Fig. 8) between one end (202 or 2004) of the stent-graft 100 with the body vessel. In the preferred embodiment, there are three sutures 400 in which each suture connects one apex of one stent hoop to two apices of another stent hoop to define a "tri-tether" connection 500. That is, my tri-tether configuration ensures that one apex (API of hoop 300f) is disposed between the two apices (AP2 and AP3 of hoop 300e) that are linked together with the suture 400, as shown here in Figures 2 and 3. The tri-tethers are preferably configured so that the middle apex AP1 of one stent hoop is aligned along an axis W-W that may be parallel to the longitudinal axis L-L with the respective apices AP1 of the other stent hoops 300e and 300f. It should be noted, however, that the implementation of the present invention is not limited to three sutures 400. Nor is one apex (e.g., AP1) of one stent hoop (e.g., 300f) is required to be disposed between two apices (e.g., AP2 and AP3) of the other stent hoop (e.g., 300e). Other configurations and orientations of the apices and the sutures are within the scope of the present invention such as, for example, the sutures 400 being located on the inner surface of the graft 200 or less than three tri-tether connections 500 being utilized.

It should be noted that the connector 400 is not required to connect to the respective apices such as that shown in Fig. 3 but can be connected at a location offset to the apices via a suitable retainer such as, for example, a hook or an eyelet and the like.

Depending on the number of sutures and the separation distance y1, y2, y3 ... so on, different radii of curvature could be attained. For example, as shown in Fig. 4, stent-graft 100 is curved along a radius of curvature R of approximately ½ of a length L1 of the stent-graft 100 (i.e., R∼ 0.5L1). In particular, the radius of curvature R defines an arcuate portion of a virtual circle such that the arcuate portion includes an included angle θ of approximately 30 to 70 degrees as measured from normal stent hoop circumference 20b (e.g., stent-graft segment S5) to the end stent-graft segment (e.g., S1). In the exemplary configuration, the radius of curvature R provides for an included angle θ of about 45 degrees where included angle θ is the sum of the included angles θ₁, θ₂, θ₃, θ₄ and so on for each stent-graft segment (i.e., S1-S4) with respect to the adjacent segment stent-graft segment. One preferred embodiment may have a radius of about 3 cm but other values can be utilized by one skilled in the art when apprised of the principles of my invention. That is, the curvature R is not limited to about 3 cm as noted here. This is due to the variations in biological anatomies. Hence, the curvature R is dependent upon the specifics of the anatomy to which an embodiment of my invention will be utilized and therefore many different sizes can be designed and utilized other than the configuration described and illustrated here.

One of the many benefits of this design is that in the constrained or compressed configuration, there is no increase in the overall profile (or thickness when the stent-graft is viewed in a side cross-sectional view) of the implant. This andadvantage is due to the combination of design features taught in this application that allow virtually no increase in the profile in the delivery stage but yet allow for a pre-configured curved once deployed in the blood vessel.

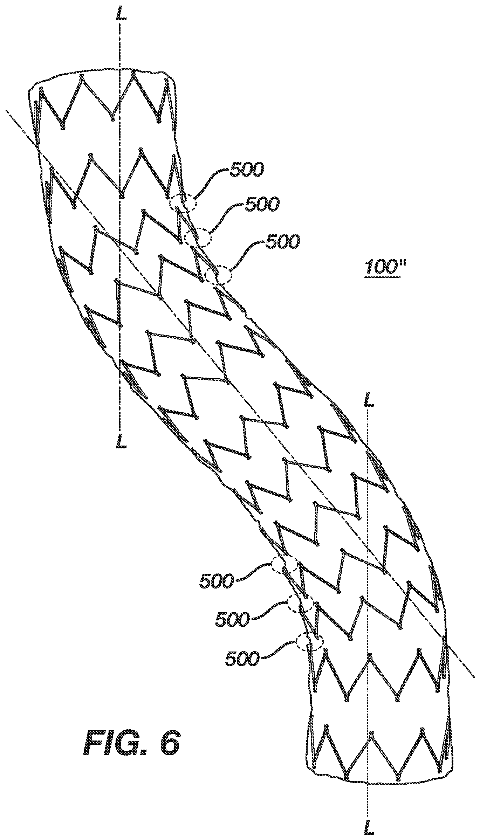

It is noted that while one curvilinear configuration is shown in Figures 1A-1C and 2-6, other curvilinear configurations can also be utilized within the scope of the present invention. For example, as shown in Figure 6, an S-curved configuration can be utilized by implementing the tri-tether connection 500 at certain locations indicated on the stent-graft 100" in Figure 6 to achieve the desired curvature. It is noted that this embodiment can be used in tortuous vessels and therefore is not limited to uses in the aorta.

It is noted that in the application of the endoprosthesis for aneurysms, the suture 400 may be a non-bioresorbable material. In other applications, suture 400 may be formed from a bioresorbable material. Suitable biodegradable materials may include polymers such as polylactic acid (i.e., PLA), polyglycolic acid (i.e., PGA), polydioxanone (i.e., PDS), polyhydroxybutyrate (i.e., PHB), polyhydroxyvalerate (i.e., PHV), and copolymers or a combination of PHB and PHV (available commercially as Biopol®), polycaprolactone (available as Capronor®), polyanhydrides (aliphatic polyanhydrides in the back bone or side chains or aromatic polyanhydrides with benzene in the side chain), polyorthoesters, polyaminoacids (e.g., poly-L-lysine, polyglutamic acid), pseudo-polyaminoacids (e.g., with back bone of polyaminoacids altered), polycyanocrylates, or polyphosphazenes. As used herein, the term "bio-resorbable" includes a suitable biocompatible material, mixture of materials or partial components of materials being degraded into other generally non-toxic materials by an agent present in biological tissue (i.e., being bio-degradable via a suitable mechanism, such as, for example, hydrolysis) or being removed by cellular activity (i.e., bioresorption, bioabsorption, or bio-resorbable), by bulk or surface degradation (i.e., bioerosion such as, for example, by utilizing a water insoluble polymer that is soluble in water upon contact with biological tissue or fluid), or a combination of one or more of the bio-degradable, bio-erodable, or bio-resorbable material noted above. In yet other applications, the suture 400 may be a shape memory material such as shape memory metal or polymers.

The suture 10 or 400 can be infused or loaded with bioactive agents to aid in the healing response or to achieve a desired physiological response. For example, bio-active agents such as blood de-clotting agent (e.g., heparin, warfarin, etc.,) anti-proliferative/antimitotic agents including natural products such as vinca alkaloids (i.e. vinblastine, vincristine, and vinorelbine), paclitaxel, epidipodophyllotoxins (i.e. etoposide, teniposide), antibiotics (dactinomycin (actinomycin D) daunorubicin, doxorubicin and idarubicin), anthracyclines, mitoxantrone, bleomycins, plicamycin (mithramycin) and mitomycin, enzymes (L-asparaginase which systemically metabolizes L-asparagine and deprives cells which do not have the capacity to synthesize their own asparagine); antiplatelet agents such as G(GP) IIb/IIIa inhibitors and vitronectin receptor antagonists; antiproliferative/antimitotic alkylating agents such as nitrogen mustards (mechlorethamine, cyclophosphamide and analogs, melphalan, chlorambucil), ethylenimines and methylmelamines (hexamethylmelamine and thiotepa), alkyl sulfonates-busulfan, nirtosoureas (carmustine (BCNU) and analogs, streptozocin), trazenes-dacarbazinine (DTIC); antiproliferative/antimitotic antimetabolites such as folic acid analogs (methotrexate), pyrimidine analogs (fluorouracil, floxuridine, and cytarabine), purine analogs and related inhibitors (mercaptopurine, thioguanine, pentostatin and 2-chlorodeoxyadenosine {cladribine}); platinum coordination complexes (cisplatin, carboplatin), procarbazine, hydroxyurea, mitotane, aminoglutethimide; hormones (i.e. estrogen); anti-coagulants (heparin, synthetic heparin salts and other inhibitors of thrombin); fibrinolytic agents (such as tissue plasminogen activator, streptokinase and urokinase), aspirin, dipyridamole, ticlopidine, clopidogrel, abciximab; antimigratory; antisecretory (breveldin); anti-inflammatory: such as adrenocortical steroids (cortisol, cortisone, fludrocortisone, prednisone, prednisolone, 6α-methylprednisolone, triamcinolone, betamethasone, and dexamethasone), non-steroidal agents (salicylic acid derivatives i.e. aspirin; para-aminophenol derivatives i.e. acetominophen; indole and indene acetic acids (indomethacin, sulindac, and etodalac), heteroaryl acetic acids (tolmetin, diclofenac, and ketorolac), arylpropionic acids (ibuprofen and derivatives), anthranilic acids (mefenamic acid, and meclofenamic acid), enolic acids (piroxicam, tenoxicam, phenylbutazone, and oxyphenthatrazone), nabumetone, gold compounds (auranofin, aurothioglucose, gold sodium thiomalate); immunosuppressives: (cyclosporine, tacrolimus (FK-506), sirolimus (rapamycin), azathioprine, mycophenolate mofetil); angiogenic agents: vascular endothelial growth factor (VEGF), fibroblast growth factor (FGF); angiotensin receptor blockers; nitric oxide donors; anti-sense oligionucleotides and combinations thereof; cell cycle inhibitors, mTOR inhibitors, and growth factor receptor signal transduction kinase inhibitors; retenoids; cyclin/CDK inhibitors; HMG co-enzyme reductase inhibitors (statins); and protease inhibitors.

All of the stent hoops described herein are substantially tubular elements that may be formed utilizing any number of techniques and any number of materials. In the preferred exemplary embodiment, all of the stent hoops are formed from a nickel-titanium alloy (Nitinol), shape set laser cut tubing.

The graft material utilized to cover all of the stent hoops may be made from any number of suitable biocompatible materials, including woven, knitted, sutured, extruded, or cast materials forming polyester, polytetrafluoroethylene, silicones, urethanes, and ultra-light weight polyethylene, such as that commercially available under the trade designation SPECTRA™. The materials may be porous or nonporous. Exemplary materials include a woven polyester fabric made from DACRON™ or other suitable PET-type polymers.

As noted above, the graft material is attached to each of the stent hoops. The graft material may be attached to the stent hoops in any number of suitable ways. In the exemplary embodiment, the graft material is attached to the stent hoops by sutures.

Depending on the stent hoops location, different types of suture knots may be utilized for retainer suture 10. Details of various embodiments of the suture knots for suture 10 or suture 400 can be found in US Patent Application Publication No. US20110071614 filed on September 24, 2009.

## Claims

1. An endovascular implant comprising:
a generally tubular graft (200) extending along a longitudinal axis (L-L) from a first opening to a second opening spaced apart along the longitudinal axis (L-L);
a plurality of stent hoops (300) attached to the graft (200) and disposed between the first opening and the second opening of the graft to define a stent graft (100), each of the stent hoops (300) having a sinusoidal configuration disposed about the longitudinal axis (L-L) with apices spaced apart along the longitudinal axis (L-L), the apices of one stent hoop (300) are spaced apart at a predetermined distance (y) along the longitudinal axis (L-L) from adjacent apices of another stent hoop (300); and
at least one suture (400) connecting one apex (AP1) of one stent hoop (300) disposed on the graft (200) to two apices (AP2, AP3) of another stent hoop (300) disposed on the graft (200) to reduce the predetermined distance (y) so that in a released configuration in a body vessel, the stent-graft is curved away from the longitudinal axis (L-L) to conform to the body vessel and reduce formation of a gap between one end of the stent-graft (100) with an inner surface of the body vessel.

2. The implant of one of claim 1, in which the at least one suture (400) comprises three sutures in which each suture (400) connects one apex (AP1) of one stent hoop (300) to two apices (AP2, AP3) of another stent hoop (300).

3. The implant of one of claim 1, in which the one apex (AP1) of one stent hoop (300) is disposed between two apices (AP2, AP3) of another stent hoop (300).

4. The implant of one of claim 1, in which the stent-graft (100) is curved along a radius of about 3 centimeters.

5. The implant of claim 4, in which the radius of curvature defines an arcuate portion of a virtual circle, wherein the arcuate portion includes an angle of approximately 45 degrees.

6. The implant of one of claim 1, in which the generally tubular graft (200) comprises a synthetic material selected from a group consisting of nylon, ePTFE, PTFE, Dacron and combinations thereof.

7. The implant of one of claim 1, in which the generally tubular graft (200) comprises a generally constant inside diameter smaller than an outside diameter of the stent hoop (300).

8. The implant of one of claim 1, in which the generally tubular graft (200) comprises at least one flared end.

9. The implant of one of claim 1, in which the plurality of stent hoops (300) are disposed on the inside surface of the stent-graft (100).

10. The implant of one of claim 1, in which the predetermined distance (y) comprises a distance selected from any value between about 1 mm to about 2 mm.

11. The implant of one of claim 1, in which another stent hoop (300) configured with retention barbs is connected to a cranial end of the graft (200).

## Patentansprüche

1. Endovaskulares Implantat, das Folgendes umfasst:
ein im Allgemeinen röhrenförmiges Transplantat (200), das sich entlang einer Längsachse (L-L) von einer ersten Öffnung zu einer zweiten Öffnung erstreckt, die entlang der Längsachse (L-L) voneinander beabstandet sind,
mehrere Stentreifen (300), die an dem Transplantat (200) befestigt und zwischen der ersten Öffnung und der zweiten Öffnung des Transplantats angeordnet sind, um ein Stenttransplantat (100) zu definieren, wobei jeder der Stentreifen (300) eine sinusförmige Konfiguration aufweist, die um die Längsachse (L-L) angeordnet ist, mit Scheiteln, die entlang der Längsachse (L-L) voneinander beabstandet sind, wobei die Scheitel eines Stentreifens (300) um eine vorbestimmte Entfernung (y) entlang der Längsachse (L-L) von benachbarten Scheiteln eines anderen Stentreifens (300) beabstandet sind, und
wenigstens eine Naht (400), die einen Scheitel (AP1) eines Stentreifens (300), der an dem Transplantat (200) angeordnet ist, mit zwei Scheiteln (AP2, AP3) eines anderen Stentreifens (300), der an dem Transplantat (200) angeordnet ist, verbindet, um die vorbestimmte Entfernung (y) zu verringern, so dass in einer gelösten Konfiguration in einem Körpergefäß das Stenttransplantat von der Längsachse (L-L) weg gekrümmt ist, um sich an das Körpergefäß anzupassen und die Bildung eines Spalts zwischen einem Ende des Stenttransplantats (100) und einer Innenfläche des Körpergefäßes zu verringern.

2. Implantat nach Anspruch 1, wobei die wenigstens eine Naht (400) drei Nähte umfasst, wobei jede Naht (400) einen Scheitel (AP1) eines Stentreifens (300) mit zwei Scheiteln (AP2, AP3) eines anderen Stentreifens (300) verbindet.

3. Implantat nach Anspruch 1, wobei der eine Scheitel (AP1) eines Stentreifens (300) zwischen zwei Scheiteln (AP2, AP3) eines anderen Stentreifens (300) angeordnet ist.

4. Implantat nach Anspruch 1, wobei das Stenttransplantat (100) entlang eines Radius von etwa 3 Zentimetern gekrümmt ist.

5. Implantat nach Anspruch 4, wobei der Krümmungsradius einen bogenförmigen Abschnitt eines virtuellen Kreises definiert, wobei der bogenförmige Abschnitt einen Winkel von annähernd 45 Grad einschließt.

6. Implantat nach Anspruch 1, wobei das im Allgemeinen röhrenförmige Transplantat (200) ein synthetisches Material umfasst, das ausgewählt ist aus einer Gruppe, die aus Nylon, ePTFE, PTFE, Dacron und Kombinationen derselben besteht.

7. Implantat nach Anspruch 1, wobei das im Allgemeinen röhrenförmige Transplantat (200) einen im Allgemeinen konstanten Innendurchmesser umfasst, der kleiner ist als ein Außendurchmesser des Stentreifens (300).

8. Implantat nach Anspruch 1, wobei das im Allgemeinen röhrenförmige Transplantat (200) wenigstens ein aufgeweitetes Ende umfasst.

9. Implantat nach Anspruch 1, wobei die mehreren Stentreifen (300) auf der Innenfläche des Stenttransplantats (100) angeordnet sind.

10. Implantat nach Anspruch 1, wobei die vorbestimmte Entfernung (y) eine Entfernung umfasst, die ausgewählt ist aus einem beliebigen Wert zwischen etwa 1 mm und etwa 2 mm.

11. Implantat nach Anspruch 1, wobei ein anderer Stentreifen (300), der mit Rückhaltewiderhaken versehen ist, mit einem kranialen Ende des Transplantats (200) verbunden ist.

## Revendications

1. Implant endovasculaire comprenant :
un greffon de forme générale tubulaire (200) qui s'étend suivant un axe longitudinal (L-L) depuis une première ouverture jusqu'à une seconde ouverture qui sont espacées l'une de l'autre suivant l'axe longitudinal (L-L) ;
une pluralité d'anneaux d'endoprothèse (300) qui sont liés sur le greffon (200) et qui sont disposés entre la première ouverture et la seconde ouverture du greffon de manière à ce qu'ils définissent un greffon formant endoprothèse (100), chacun des anneaux d'endoprothèse (300) présentant une configuration sinusoïdale qui est disposée autour de l'axe longitudinal (L-L), des apex afférents étant espacés les uns des autres suivant l'axe longitudinal (L-L), les apex d'un anneau d'endoprothèse (300) étant espacés d'une distance prédéterminée (y) suivant l'axe longitudinal (L-L) vis-à-vis d'apex adjacents d'un autre anneau d'endoprothèse (300) ; et
au moins une suture (400) qui connecte un apex (AP1) d'un anneau d'endoprothèse (300) qui est disposé sur le greffon (200) à deux apex (AP2, AP3) d'un autre anneau d'endoprothèse (300) qui est disposé sur le greffon (200) de manière à réduire la distance prédéterminée (y) de telle sorte que dans une configuration libérée à l'intérieur d'un vaisseau du corps, le greffon formant endoprothèse soit éloigné par incurvation par rapport à l'axe longitudinal (L-L) de manière à ce qu'il se conforme au vaisseau du corps et de manière à ce qu'il réduise la formation d'un espace entre une extrémité du greffon formant endoprothèse (100) et une surface interne du vaisseau du corps.

2. Implant selon la revendication 1, dans lequel l'au moins une suture (400) comprend trois sutures, chaque suture (400) connectant un apex (AP1) d'un anneau d'endoprothèse (300) à deux apex (AP2, AP3) d'un autre anneau d'endoprothèse (300).

3. Implant selon la revendication 1, dans lequel l'apex considéré (AP1) d'un anneau d'endoprothèse (300) est disposé entre deux apex (AP2, AP3) d'un autre anneau d'endoprothèse (300).

4. Implant selon la revendication 1, dans lequel le greffon formant endoprothèse (100) est incurvé selon un rayon d'environ 3 centimètres.

5. Implant selon la revendication 4, dans lequel le rayon de courbure définit une section en forme d'arc d'un cercle virtuel, dans lequel la section en forme d'arc inclut un angle d'approximativement 45 degrés.

6. Implant selon la revendication 1, dans lequel le greffon de forme générale tubulaire (200) comprend un matériau synthétique qui est sélectionné parmi un groupe qui est constitué par le nylon, l'ePTFE, le PTFE, le Dacron et des combinaisons de ceux-ci.

7. Implant selon la revendication 1, dans lequel le greffon de forme générale tubulaire (200) présente un diamètre intérieur généralement constant qui est inférieur à un diamètre extérieur de l'anneau d'endoprothèse (300).

8. Implant selon la revendication 1, dans lequel le greffon de forme générale tubulaire (200) comprend au moins une extrémité évasée.

9. Implant selon la revendication 1, dans lequel les anneaux d'endoprothèse de la pluralité d'anneaux d'endoprothèse (300) sont disposés sur la surface intérieure du greffon formant endoprothèse (100).

10. Implant selon la revendication 1, dans lequel la distance prédéterminée (y) comprend une distance qui est sélectionnée parmi n'importe quelles valeurs entre environ 1 environ et environ 2 mm.

11. Implant selon la revendication 1, dans lequel un autre anneau d'endoprothèse (300) qui est configuré de manière à ce qu'il comporte des ardillons de retenue est connecté à une extrémité crânienne du greffon (200).
